# EUROPEAN PATENT APPLICATION

(11) **EP 4 582 055 A1**
(43) Date of publication of application: **09.07.2025**
(21) Application number: 23859340.4
(22) Date of filing: 29.08.2023
(51) Int. Cl.: A61F 2/24

(54) **PROSTHESIS SYSTEM AND DELIVERY APPARATUS**

(30) Priority: 29.08.2022 CN 202211057484
(71) Applicant: Enlight Medical Technologies (Shanghai) Co., Ltd., Shanghai 201318 (CN)
(72) Inventor: ZHOU, Guanguan, Shanghai 201315 (CN); LI, Fang, Shanghai 201315 (CN); HU, Zhanming, Shanghai 201315 (CN); LI, Wensi, Shanghai 201315 (CN)
(74) Representative: Balder IP Law, S.L.
(86) International application number: PCT/CN2023/115492
(87) International publication number: WO 2024/046310

(57) **Abstract**

Provided is a prosthesis system. The prosthesis system includes a delivery apparatus (100) and a prosthesis. The delivery apparatus (100) includes a connecting shaft (110) and a control wire (120). The prosthesis includes a base (210) and a first movable component. A preset channel (300) is provided in at least one of the connecting shaft (110) and the base (210). The control wire (120) includes a first segment (121), a second segment (122) and a third segment (123) connected successively. At least a part of each of the first segment (121) and the third segment (123) connected to the second segment (122) has a size larger than a size of the second segment (122). The second segment (122) is accommodatable within the preset channel (300). In response to the preset channel (300) being in the first state, the first segment (121) and the third segment (123) are configured to cause the second segment (122) to be constrained within the preset channel (300), and in response to the preset channel (300) being in the second state, the control wire (120) is separable from the preset channel (300). In the prosthesis system and delivery apparatus in the embodiments of the present disclosure, the movable component of the prosthesis can be safely and reliably adjusted by the delivery apparatus (100), thereby reducing the surgical risk.

## Description

### CROSS REFERENCE TO RELATED APPLICATIONS

This application is based on and claims priority to Chinese Patent Application No. CN202211057484.4, filed on August 29, 2022, which is incorporated by reference herein in its entirety.

### TECHNICAL FIELD

The present disclosure relates to the technical field of medical devices, and in particular, to a prosthesis system and a delivery apparatus.

### BACKGROUND

With the development and progress of science and technology, minimally invasive surgery has been applied to various fields of surgery. Minimally invasive surgery has been welcomed by patients due to its advantages, such as less trauma, less pain, and faster recovery.

In an existing minimally invasive surgery, a delivery apparatus is generally used to deliver a prosthesis to a target location in the human body through a surgical path such as a natural lumen of the human body, then a control wire movably connected to a prosthesis is utilized to adjust a state of the prosthesis, and finally the purpose of the surgery is achieved by implanting the prosthesis in the human body.

U.S. Patent Document US20210145574A1 discloses a method for performing minimally invasive surgery, in which one end of a control wire has a bump, and the other end of the control wire passes through a connecting shaft of a delivery apparatus, is movably connected to a prosthesis and then extends to a proximal end of the delivery apparatus. In this case, through configuration of the connecting shaft of the delivery apparatus and a center rod, the bump is restricted by the connecting shaft and a center rod of the delivery apparatus, so that the bump cannot pass through the connecting shaft in an extension direction of the control wire. This allows an operator to operate, at the proximal end of the delivery apparatus, an end of the control wire away from the bump to adjust a state of the prosthesis.

However, during the surgery process, the bump of the control wire may easily run out of the connecting shaft in a direction opposite to the extension direction of the control wire, which makes the control wire easy to be jammed on an other component of the delivery apparatus or certain organs inside the human body, and thus increases the surgical risk.

### SUMMARY

The present disclosure provides a prosthesis system and a delivery apparatus, in which the movable component of the prosthesis can be safely and reliably adjusted by the delivery apparatus, thereby reducing the surgical risk.

Some embodiments of the present disclosure provide a prosthesis system. The prosthesis system includes a delivery apparatus and a prosthesis. The delivery apparatus includes a connecting shaft and a control wire. The prosthesis includes a base and a first movable component, where the base is detachably coupled to the connecting shaft. A preset channel is provided in at least one of the connecting shaft and the base at an angle relative to an axis of the connecting shaft. The control wire includes a first segment, a second segment and a third segment connected successively, at least a part of each of the first segment and the third segment connected to the second segment has a size larger than a size of the second segment, the first segment is configured to independently drive the first movable component, and the second segment is accommodatable within the preset channel. The preset channel has a first state and a second state, in response to the preset channel being in the first state, the first segment and the third segment are configured to cause the second segment to be constrained within the preset channel, and in response to the preset channel being in the second state, the control wire is separable from the preset channel.

Some embodiments of the present disclosure provide a delivery apparatus configured to deliver a prosthesis. The prosthesis includes a base and a first movable component, and the delivery apparatus includes a connecting shaft and a control wire. The connecting shaft is detachably coupled to the base, where a preset channel is provided in the connecting shaft at an angle relative to an axis of the connecting shaft, or provided at a coupling site between the connecting shaft and the base at an angle relative to an axis of the connecting shaft. The control wire includes a first segment, a second segment and a third segment connected successively, at least a part of each of the first segment and the third segment connected to the second segment has a size larger than a size of the second segment, the first segment is configured to independently drive the first movable component, and the second segment is accommodatable within the preset channel. The preset channel has a first state and a second state, in response to the preset channel being in the first state, the first segment and the third segment are configured to cause the second segment to be constrained within the preset channel, and in response to the preset channel being in the second state, the control wire is separable from the preset channel.

According to the prosthesis system and delivery apparatus in some embodiments of the present disclosure, during the minimally invasive implantation procedure, the preset channel is set to the first state to constrain the second segment of the control wire within the preset channel by the first segment and the third segment of the control wire, and then after the prosthesis is delivered by the connecting shaft to a target position in the human body via a surgical path such as a natural lumen of the human body, the first movable component can be adjusted by the first segment of the control wire so that the state of the prosthesis is changed to achieve the implantation of the prosthesis. When it is necessary to separate the delivery apparatus from the prosthesis, the preset channel is set to the second state, and in the second state, the control wire can separate from the preset channel, which facilitates separation of the control wire from the prosthesis and then withdrawal of the control wire from the human body. At this time, the base of the prosthesis can also be separated from the connecting shaft of the delivery apparatus, thereby facilitating the withdrawal of the connecting shaft from the human body to complete the implantation procedure. In this way, in the delivery apparatus of the prosthesis system, the movable component of the prosthesis can be safely and reliably adjusted to achieve a change in state of the prosthesis, making the prosthesis system safer so that the surgical procedures are safer and more reliable.

In addition, the preset channel in the prosthesis system provided in the present disclosure has the first state and the second state to work in conjunction the control wire to accomplish the relevant surgical procedures. That is, when the preset channel is in the first state, the first segment and the third segment are configured to cause the second segment to be constrained within the preset channel so that the second segment always remains within the preset channel and cannot move relative to the preset channel, and the part of each of the first segment and the third segment connected to the second segment cannot be moved relative to the preset channel, so as to prevent the first segment and the third segment from running out as in the existing art, and thus avoid the problem in the existing art that "the control wire is prone to be jammed on an other component of the delivery apparatus or certain organs in the human body", thereby reducing the surgical risk.

In some embodiments, in response to the preset channel being in the first state, a part of the preset channel configured to accommodate the second segment has a first size, and the first size is smaller than the size of the part of each of the first segment and the third segment connected to the second segment and larger than or equal to the size of the second segment; and in response to the preset channel being in the second state, the part of the preset channel configured to accommodate the second segment has a second size, and the second size is larger than a size of each of at least one of the first segment and the third segment.

In some embodiments, the delivery apparatus further includes a center rod. At least one of the connecting shaft and the base is provided with an accommodating channel arranged in an axial direction of the connecting shaft, where the accommodating channel intersects the preset channel, and an area where the accommodating channel overlaps the preset channel is an overlap area. When the center rod is placed into the accommodating channel and occupies at least a part of the overlap area, the preset channel is in the first state, and a size of a part of the remaining area of the preset channel configured to accommodate the second segment is the first size. When the center rod is positioned outside the overlap area, the preset channel is in the second state, and a size of the part of the preset channel configured to accommodate the second segment is the second size.

In some embodiments, a size of a part of the overlap area occupied by the center rod is a third size, the second size is larger than or equal to a sum of the third size and the size of the second segment, and the second size is smaller than a sum of the third size and the size of the part of each of the first segment and the third segment connected to the second segment.

In some embodiments, the preset channel is arranged in the connecting shaft, the accommodating channel includes a first accommodating segment arranged in the connecting shaft, and an area where the accommodating segment overlaps the preset channel is the overlap area. When the center rod is placed into the first accommodating segment and occupies at least the part of the overlap area, the preset channel is in the first state.

In some embodiments, the preset channel is arranged in the base, the accommodating channel includes a first accommodating segment on the connecting shaft, and a second accommodating segment on the base. The first accommodating segment and the second accommodating segment are connected with each other when the base is detachably coupled to the connecting shaft, and an area where the second accommodating segment overlaps the preset channel is the overlap area. When the center rod is placed into the first accommodating segment and the second accommodating segment and occupies at least the part of the overlap area, the preset channel is in the first state.

In some embodiments, the preset channel is arranged at a coupling site between the connecting shaft and the base, and the accommodating channel and the preset channel overlap at the coupling site to form the overlap area.

In some embodiments, in response to a size of the overlap area being equal to the second size, the center rod occupies a part of the overlap area; and in response to the size of the overlap area being smaller than the second size, the center rod occupies at least a part of the overlap area.

In some embodiments, the preset channel is arranged at a coupling site between the connecting shaft and the base. When the connecting shaft is coupled to the base, the preset channel is in the first state and is closed. When the connecting shaft is detached from the base, the preset channel is in the second state and is non-closed.

In some embodiments, the connecting shaft has a first contact surface, where the first contact surface is provided with a first through-slot. The base has a second contact surface configured to match the first contact surface, where the second contact surface is provided with a second through-slot. When the connecting shaft is coupled to the base, the first contact surface and the second contact surface fit together, and the first through-slot and the second through-slot together form the preset channel that is closed. When the connecting shaft is detached from the base, the first contact surface and the second contact surface are separated from each other so that the preset channel is non-closed.

In some embodiments, the first contact surface includes a first distal blocking surface, a first inclined surface, and a first proximal blocking surface arranged in sequence from distal to proximal, and a maximum vertical distance from an edge of the connecting shaft to the first inclined surface becomes smaller as an axial distance from the first inclined surface to the first distal blocking surface becomes larger. The second contact surface includes a second distal blocking surface, a second inclined surface and a second proximal blocking surface arranged in sequence from distal to proximal, and a maximum vertical distance from an edge of the base to the second inclined surface becomes smaller as an axial distance from the second inclined surface to the second proximal blocking surface becomes larger. The first through-slot is arranged on the first inclined surface, and the second through-slot is correspondingly arranged on the second inclined surface. When the connecting shaft is coupled to the base, the first through-slot and the second through-slot together form the preset channel that is closed, and when the connecting shaft is detached from the base, the first through-slot is separated from the second through-slot so that the preset channel is non-closed.

In some embodiments, the connecting shaft has a first contact surface, the base has a second contact surface configured to match the first contact surface, and one of the first contact surface and the second contact surface is provided with a third through-slot. When the connecting shaft is coupled to the base, the first contact surface and the second contact surface fit together, and a slot wall of the third through-slot and a corresponding part of the other one of the first contact surface and the second contact surface together form the preset channel that is closed. When the connecting shaft is detached from the base, the first contact surface and the second contact surface are separated from each other so that the preset channel is non-closed.

In some embodiments, the first contact surface includes a third distal blocking surface, a third inclined surface, and a third proximal blocking surface arranged in sequence from distal to proximal, and a maximum vertical distance from an edge of the connecting shaft to the third inclined surface becomes smaller as an axial distance from the third inclined surface to the third distal blocking surface becomes larger. The second contact surface includes a fourth distal blocking surface, a fourth inclined surface, a transition surface, and a fourth proximal blocking surface arranged in sequence from distal to proximal, and a maximum vertical distance from an edge of the base to the fourth inclined surface becomes smaller as an axial distance from the fourth inclined surface to the fourth proximal blocking surface becomes larger. The third through-slot is arranged on the third inclined surface to correspond to the transition surface. When the connecting shaft is coupled to the base, the third through-slot and the transition surface together form the preset channel that is closed, and when the connecting shaft is detached from the base, the third through-slot and the transition surface are separated from each other so that the preset channel is non-closed.

In some embodiments, the first contact surface includes a third distal blocking surface, a third inclined surface, a transition surface, and a third proximal blocking surface arranged in sequence from distal to proximal, and a maximum vertical distance from an edge of the connecting shaft to the third inclined surface becomes smaller as an axial distance from the third inclined surface to the third distal blocking surface becomes larger. The second contact surface includes a fourth distal blocking surface, a fourth inclined surface, and a fourth proximal blocking surface arranged in sequence from distal to proximal, and a maximum vertical distance from an edge of the base to the fourth inclined surface becomes smaller as an axial distance from the fourth inclined surface to the fourth proximal blocking surface becomes larger. The third through-slot is arranged on the fourth inclined surface to correspond to the transition surface. When the connecting shaft is coupled to the base, the third through-slot and the transition surface together form the preset channel that is closed, and when the connecting shaft is detached from the base, the third through-slot and the transition surface are separated from each other so that the preset channel is non-closed.

In some embodiments, the prosthesis further includes a second movable component, and the third segment is configured to independently drive the second movable component.

In some embodiments, the prosthesis is a mitral valve clip or a tricuspid valve clip.

In some embodiments, the first movable component is a first gripper, the second movable component is a second gripper, and the first gripper and the second gripper are rotatable about an axis of the base. The first segment is movably connected to the first gripper, and the second segment is movably connected to the second gripper.

### BRIEF DESCRIPTION OF THE DRAWINGS

FIG. 1 is a schematic structure view illustrating coupling between a delivery apparatus at a distal end and a mitral valve clip according to an embodiment of the present disclosure.
FIG. 2 is a schematic structure view illustrating a gripper of the mitral valve clip coupled with the delivery device provided in an embodiment of the present disclosure during movement.
FIG. 3 is a schematic structure view of a control wire according to an embodiment of the present disclosure.
FIG. 4 is a partially enlarged view of part a in FIG. 3.
FIG. 5 is a longitudinal cross-sectional view illustrating the cooperation of a control wire, a center rod and a preset channel according to an embodiment of the present disclosure.
FIG. 6 is a schematic structure view illustrating the cooperation of a center rod and a preset channel according to an embodiment of the present disclosure.
FIG. 7 is a longitudinal cross-sectional view illustrating the cooperation of a control wire, a center rod and a preset channel according to another embodiment of the present disclosure.
FIG. 8 is a transverse cross-sectional view illustrating the cooperation of a control wire, a center rod and a preset channel according to an embodiment of the present disclosure.
FIG. 9 is a transverse cross-sectional view illustrating the cooperation of a control wire, a center rod and a preset channel according to another embodiment of the present disclosure.
FIG. 10 is a longitudinal schematic view of a connecting shaft cooperating with a base to form a preset channel according to an embodiment of the present disclosure.
FIG. 11 is a longitudinal schematic view of a connecting shaft cooperating with a base to form a preset channel according to another embodiment of the present disclosure.
FIG. 12 is a longitudinal cross-sectional view of a connecting shaft cooperating with a base to form a preset channel according to another embodiment of the present disclosure.
FIG. 13 is a brief schematic view of a mitral valve clip clamping mitral valve leaflets according to an embodiment of the present disclosure.
FIG. 14 is a schematic structure view of a gripper cooperating with a control wire according to an embodiment of the present disclosure.
FIG. 15 is a schematic structure view of a gripper cooperating with a control wire according to another embodiment of the present disclosure.
FIG. 16 is a schematic structure view of a gripper cooperating with a control wire according to another embodiment of the present disclosure.

### DETAILED DESCRIPTION OF THE EMBODIMENTS

It is to be understood by those of ordinary skill in the art that in various embodiments of the present disclosure, many technical details are presented in order to provide the reader with a better understanding of the present disclosure. However, technical solutions claimed to be protected by the present disclosure can also be achieved based on various variations and modifications of the following embodiments.

In various embodiments of the present disclosure, a "proximal end" or "proximal side" refers to an end closer to an operator and away from a patient, and accordingly, a "distal end" or "distal side" refers to an end closer to the patient and away from the operator. In the various embodiments of the present disclosure, the term "parallel" or "perpendicular" should not be narrowly understood as 0° or 90°, but should be understood as referring to a reasonable angle range that includes 0° or 90°, with some degree of fluctuation, under the premise of achieving the technical effect.

The embodiments of the present disclosure provide a prosthesis system. The prosthesis system includes a delivery apparatus and a prosthesis. The delivery apparatus includes a connecting shaft and a control wire. The prosthesis includes a base and a first movable component, where the base is detachably coupled to the connecting shaft. A preset channel is provided in at least one of the connecting shaft and the base at an angle relative to an axis of the connecting shaft. The control wire includes a first segment, a second segment and a third segment connected successively, at least a part of each of the first segment and the third segment connected to the second segment has a size larger than a size of the second segment, the first segment is configured to independently drive the first movable component, and the second segment is accommodatable within the preset channel. The preset channel has a first state and a second state, in response to the preset channel being in the first state, the first segment and the third segment are configured to cause the second segment to be constrained within the preset channel, and in response to the preset channel being in the second state, the control wire is separable from the preset channel.

According to the prosthesis system in some embodiments of the present disclosure, during a minimally invasive implantation procedure, the preset channel is set to the first state to constrain the second segment of the control wire within the preset channel by the first segment and the third segment of the control wire, and then after the prosthesis is delivered by the connecting shaft to a target position in the human body via a surgical path such as a natural lumen of the human body, the first movable component can be adjusted by the first segment of the control wire so that the state of the prosthesis is changed to achieve the implantation of the prosthesis. When it is necessary to separate the delivery apparatus from the prosthesis, the preset channel is set to the second state, and in the second state, the control wire can be separated from the preset channel, which facilitates separation of the control wire from the prosthesis and then withdrawal of the control wire from the human body. At this time, the base of the prosthesis can also be separated from the connecting shaft of the delivery apparatus, thereby facilitating the withdrawal of the connecting shaft from the human body to complete the implantation procedure. In this way, in the delivery apparatus of the prosthesis system, the movable component of the prosthesis can be safely and reliably adjusted to achieve a change in state of the prosthesis, making the prosthesis system safer so that the surgical procedures are safer and more reliable.

In addition, when the preset channel is in the first state, the first segment and the third segment are configured to cause the second segment to be constrained within the preset channel so that the second segment always remains within the preset channel and cannot move relative to the preset channel, and the part of each of the first segment and the third segment connected to the second segment cannot be moved relative to the preset channel, so as to prevent the first segment and the third segment from running out as in the existing art, and thus avoid the technical problem in the existing art that "the control wire is prone to be jammed on an other component of the delivery apparatus or certain organs in the human body", thereby reducing the surgical risk.

In order to make the purpose, technical solutions and advantages of the present disclosure clearer, embodiments of the present disclosure will be described in detail below in conjunction with the accompanying drawings.

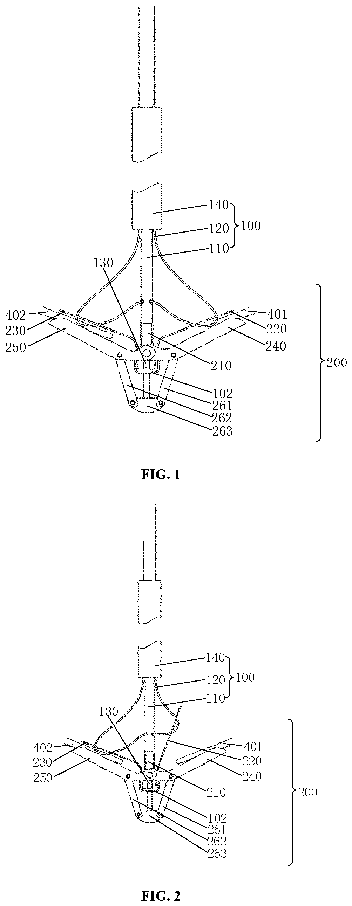

Referring to FIGS. 1 and 19, some embodiments of the present disclosure provide a prosthesis system including a delivery apparatus 100 and a prosthesis. The delivery apparatus 100 includes a connecting shaft 110 and a control wire 120. The prosthesis includes a base 210 and a first movable component. The base 210 is detachably coupled to the connecting shaft 110. A preset channel 300 is provided in at least one of the connecting shaft 110 and the base 210 at an angle relative to an axis of the connecting shaft 110. The control wire 120 includes a first segment 121, a second segment 122 and a third segment 123 connected successively. At least a part of each of the first segment 121 and the third segment 123 connected to the second segment 122 has a size larger than a size of the second segment 122. The first segment 121 is configured to independently drive the first movable component. The second segment 122 is accommodatable within the preset channel 300. The preset channel 300 has a first state and a second state. In response to the preset channel 300 being in the first state, the first segment 121 and the third segment 123 are configured to cause the second segment 122 to be constrained within the preset channel 300, and in response to the preset channel 300 being in the second state, the control wire 120 is separable from the preset channel 300.

"The preset channel 300 being provided in the at least one of the connecting shaft 110 and the base 210 at the angle relative to the axis of the connecting shaft 110" disclosed herein refers to that an extension direction of the preset channel 300 (i.e., an axis of the preset channel 300) has an angle larger than 0° and smaller than 180° with respect to the axis of the connecting shaft 110, and the preset channel 300 is arranged in the at least one of the connecting shaft 110 and the base 210. In some embodiments, the extension direction of the preset channel 300 is perpendicular to the axis of the connecting shaft 110. When the base 210 is coupled to the connecting shaft 110, the axis of the connecting shaft 110 is co-linear with an axis of the base 210. Thus, when the preset channel 300 is disposed in the base 210 or between the base 210 and the connecting shaft 110, the preset channel 300 being disposed at the angle relative to the axis of the connecting shaft 110 means that the preset channel 300 is disposed at an angle relative to the axis of the base 210.

"At least the part of each of the first segment 121 and the third segment 123 connected to the second segment 122 having the size larger than the size of the second segment 122" refers to that a part of the first segment 121 connected to the second segment 122 has a size larger than the size of the second segment 122, and a part of the third segment 123 connected to the second segment 122 has a size larger than the size of the second segment 122. The size herein refers to a cross-sectional size of the control wire 120 in a direction perpendicular to an extension direction of the control wire 120. For example, the size of the second segment 122 refers to a cross-sectional size of the second segment 122 in a direction perpendicular to an extension direction of the second segment 122. In a case where the control wire 120 is circular in cross-section, a size of the control wire 120 is a diameter of the cross-section of the control wire 120. For example, the size of the second segment 122 is a diameter of the second segment 122. In a case where the control wire 120 is non-circular in cross-section, the size of the control wire 120 is a size defined by a specific rule, e.g., the size of the control wire 120 is twice a distance from a geometric center of the cross-section of the control wire 120 to a point on an edge of the cross-section of the control wire 120. For example, the size of the second segment 122 is a diameter of a circumscribed circle of the cross-section of the second section 122.

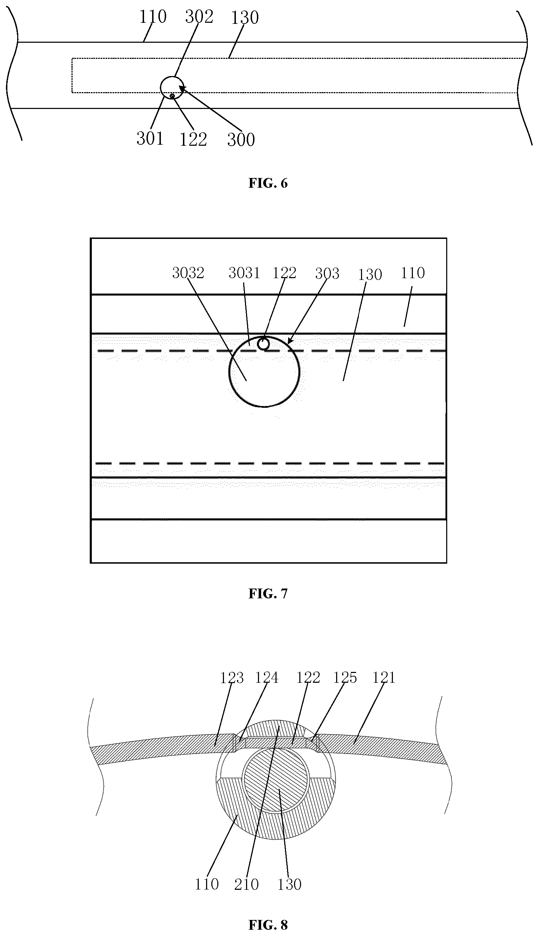

As shown in FIG. 8, in some embodiments, to facilitate the manufacture of the control wire 120, each of the first segment 121 and the third segment 123 has the same size at any portion. That is, a size of the part of the first segment 121 connected to the second segment 122 is equal to a size of the rest part of the first segment 121, and a size of the part of the third segment 123 connected to the second segment 122 is equal to a size of the rest part of the third segment 123. In some embodiments, the second segment 122 may also have the same size at any portion.

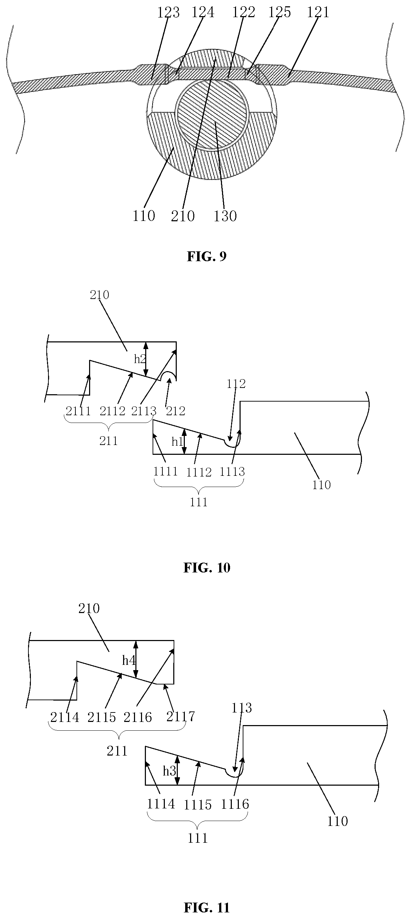

As shown in FIG. 9, in yet further embodiments, the part of the first segment 121 connected to the second segment 122 has a size larger than the size of the second segment 122, and the rest part of the first segment 121 has a size smaller than the size of the part of the first segment 121 connected to the second segment 122. In other embodiments, the part of the third segment 123 connected to the second segment 122 has a size larger than the size of the second segment 122, and the rest part of the third segment 123 has a size smaller than the size of the part of the third segment 123 connected to the second segment 122.

In some embodiments, the first movable component is movably disposed on the base 210. The first movable component being movably disposed on the base 210 refers to that the first movable component is directly movably connected to the base 210, or the first movable component is movably coupled to the base 210 through an other component, which is not limited in the present disclosure.

In some embodiments, since it is necessary to control the movement of the first movable component by the first segment 121, the control wire 120 is prepared from a material that is rigid and has a higher modulus of elasticity, such as a medical stainless steel and a titanium alloy.

In some embodiments, the preset channel 300 and the control wire 120 are configured in size such that in response to the preset channel 300 being in the first state, the first segment 121 and the third segment 123 cannot pass through the preset channel 300 and can only be adjacent to the preset channel 300, while the second segment 122 is constrained within the preset channel 300; and in response to the preset channel 300 being in the second state, the control wire 120 is separable from the preset channel 300.

In some embodiments, a size of the preset channel 300 in the first state is different from a size of the preset channel 300 in the second state. For example, in response to the preset channel 300 being in the first state, a part of the preset channel 300 configured to accommodate the second segment 122 has a first size, and the first size is smaller than the size of the part of each of the first segment 121 and the third segment 123 connected to the second segment 122 and larger than or equal to the size of second segment 122. In response to the preset channel 300 being in the second state, the part of the preset channel 300 configured to accommodate the second segment 122 has a second size, and the second size is larger than a size of each of at least one of the first segment 121 and the third segment 123.

In some embodiments, "the first size of the part of the preset channel 300 in the first state configured to accommodate the second segment 122" refers to a cross-sectional size of the part of the preset channel 300 in the first state configured to accommodate the second segment 122 of the control wire 120 in a direction perpendicular to the extension direction of the preset channel 300. In other embodiments, "the second size of the part of the preset channel 300 in the second state configured to accommodate the second segment 122" refers to a cross-sectional size of the part of the preset channel 300 in the second state configured to accommodate the second segment 122 of the control wire 120 in the direction perpendicular to the extension direction of the preset channel 300.

The first size of the preset channel 300 and the size of the control wire 120 are configured such that the first size is smaller than the size of the part of each of the first segment 121 and the third segment 123 connected to the second segment 122 and larger than or equal to the size of second segment 122. Thus, the size of the second segment 122 is smaller than or equal to the first size so that the second segment 122 can be accommodated within the preset channel 300 in the first state. Meanwhile, the size of the part of the first segment 121 connected to the second segment 122 is larger than the first size so that the first segment 121 cannot pass through the preset channel 300 in the first state, and the size of the part of the third segment 123 connected to the second segment 122 is larger than the first size so that the third segment 123 cannot pass through the preset channel 300 in the first state, which in turn causes the second segment 122 accommodated within the preset channel 300 to be constrained within the preset channel.

The second size of the preset channel 300 and the size of the control wire 120 are configured such that the second size is larger than or equal to the size of each of at least one of the first segment 121 and the third segment 123. Thus, the size of the first segment 121 at any portion in the extension direction of the first segment 121 is smaller than the second size, and/or the size of the third segment 123 at any portion in the extension direction of the third segment 123 is smaller than the second size, so that the control wire 120 can be moved in the preset channel 300 in the second state and thus is separable from the preset channel 300.

In this way, it can be achieved that in response to the preset channel 300 being in the first state, the first segment 121 and the third segment 123 cannot pass through the preset channel 300, and the second segment 122 is constrained within the preset channel 300; and in response to the preset channel 300 being in the second state, the preset channel 300 allows the control wire 120 to be separable from the preset channel 300.

In some embodiments, the second segment 122 has the same length as the preset channel 300. In this way, an end of each of the first segment 121 and the third segment 123 connected to the second segment 122 is disposed adjacent to a respective port of the preset channel so that the first segment 121 and the third segment 123 are trapped outside two ends of the preset channel 300 respectively, which facilitates precise control of the movable component of the prosthesis by the control wire 120. In this case, the preset channel 300 is configured to only accommodate the second segment 122.

In some embodiments, the second segment 122 may have a length smaller than a length of preset channel 300. In this case, a junction between the second segment 122 and at least one of the first segment 121 and the third segment 123 may be within the preset channel 300. That is, the preset channel 300 accommodates the second segment 122 and a part of each of the at least one of the first segment 121 and the third segment 123.

In some embodiments, the delivery apparatus 100 further includes a center rod 130, and the size of the preset channel 300 is adjusted by controlling whether the center rod 130 overlaps the preset channel 300. For example, at least one of the connecting shaft 110 and the base 210 is provided with an accommodating channel arranged in an axial direction of the connecting shaft 110, which intersects the preset channel 300. An overlap area is defined by an area where the accommodating channel overlaps the preset channel 300. When the center rod 130 is placed into the accommodating channel and occupies at least a part of the overlap area, the preset channel 300 is in the first state, and a size of a part of the remaining area of the preset channel 300 configured to accommodate the second segment 122 is the first size. When the center rod 130 is positioned outside the overlap area, the preset channel 300 is in the second state, and a size of the part of the preset channel 300 configured to accommodate the second segment is the second size. In some embodiments, when the center rod 130 is located at the coupling site of the connecting shaft 110 to the base 210, the connecting shaft 110 and the base 210 remain at least radially stationary relative to each other.

In this way, it is possible to make the second size larger than the first size, thereby facilitating the condition that the first size is smaller than the size of the part of each of the first segment 121 and the third segment 123 connected to the second segment 122 and larger than or equal to the size of second segment 122, and the second size is larger than the size of each of at least one of the first segment 121 and the third segment 123.

In some embodiments, the preset channel 300 may be switched from the first state to the second state. Furthermore, when it is necessary to switch the preset channel 300 from the first state to the second state, this can be accomplished simply by placing the center rod 130 outside the overlap area.

In some embodiments, a size of a part of the overlap area occupied by the center rod 130 is a third size, the second size is larger than or equal to a sum of the third size and the size of the second segment 122, and the second size is smaller than a sum of the third size and the size of the part of each of the first segment 121 and the third segment 123 connected to the second segment 122.

In some embodiments, the preset channel 300 is arranged in the connecting shaft 110, and the accommodating channel includes a first accommodating segment 410 arranged in the connecting shaft 110. An area where the accommodating segment 410 overlaps the preset channel 300 defines the overlap area. When the center rod 130 is placed into the first accommodating segment 410 and occupies at least a part of the overlap area, the preset channel 300 is in the first state.

In some embodiments, a size of the overlap area is smaller than the second size.

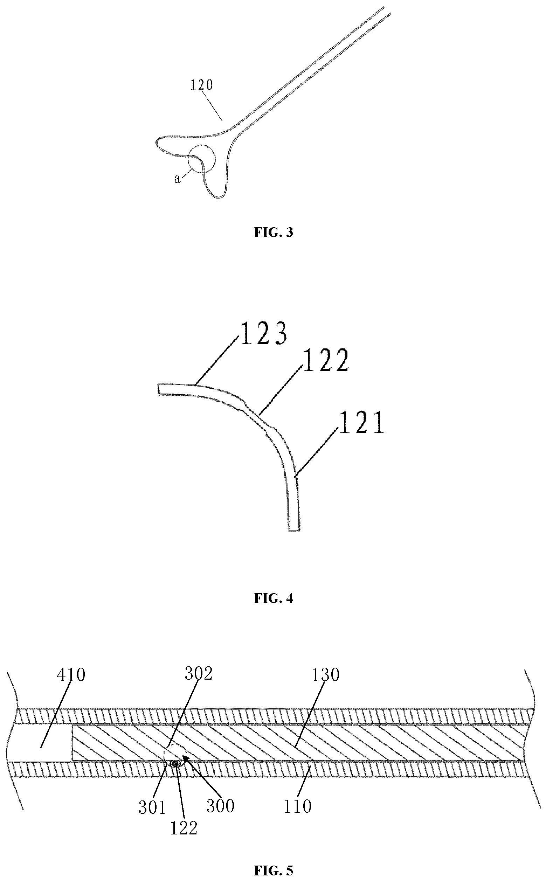

Referring to FIGS. 5 and 6, in one example, the accommodating channel includes the first accommodating segment 410 arranged in the connecting shaft 110 to accommodate the center rod 130. The preset channel 300 is arranged in the connecting shaft 110, and the preset channel 300 forms a passage slot 301 in an inner wall of the connecting shaft 110, and also forms a first wall hole 302 opposite the passage slot 301 on the connecting shaft 110. The first accommodating segment 410 partially overlaps the preset channel 300, and an area defined by the first wall hole 302 is the overlap area. When the center rod 130 is placed into the accommodating channel and occupies at least a part of the overlap area so that the preset channel 300 is in the first state, a space of the preset channel 300 includes at least a space defined by the passage slot 301 and a part of an outer surface of the center rod 130 facing the passage slot 301. When the center rod 130 does not occupy the overlap area so that the preset channel 300 is in the second state, a space of the preset channel 300 is defined by the passage slot 301 and the first wall hole 302 and can accommodate the control wire 120.

In some embodiments, the size of the overlap area is equal to the second size.

Referring to FIG. 7, in one example, also similar to the embodiments described above, the accommodating channel includes a first accommodating segment 410 arranged in the connecting shaft 110 to accommodate the center rod 130. The preset channel 300 is arranged in the connecting shaft 110. The difference is that the preset channel 300 forms a second wall hole 303 on the connecting shaft 110, and the second wall hole 303 passes through the connecting shaft 110. The first accommodating segment 410 overlaps the preset channel 300, and an area defined by the second wall hole 303 is an overlap area. A size of the center rod 130 is smaller than a size of the first wall accommodating segment 410, so the center rod 130 can only occupy a part of the overlap area. As a result, the second wall hole 303 is divided into a first hole wall portion 3032 corresponding to a space that can be occupied by the center rod 130 and a second hole wall portion 3031 corresponding to a space that cannot be occupied by the center rod 130. When the center rod 130 is placed into the first accommodating segment 410 and occupies a part of the overlap area so that the preset channel 300 is in the first state, a space of the preset channel 300 is defined by the second hole wall portion 3031 and a corresponding part of the outer surface of the center rod 130. When the center rod 130 does not occupy the overlap area so that the preset channel 300 is in the second state. In this case, both the first hole wall portion 3032 and the second hole wall portion 3031 can accommodate the control wire 120, and the space of the preset channel 300 is defined by the first hole wall portion 3032 and the second hole wall portion 3031, i.e., the second wall hole 303.

In some embodiments, the preset channel 300 may also be arranged in the base 210. The accommodating channel includes a first accommodating segment 410 arranged in the connecting shaft 110, and a second accommodating segment arranged in the base 210. The first accommodating segment 410 and the second accommodating segment are connected with each other when the base 210 is detachably coupled to the connecting shaft 110, and an area where the second accommodating segment overlaps the preset channel 300 is the overlap area. When the center rod 130 is placed into the first accommodating segment 410 and the second accommodating segment and occupies at least a part of the overlap area, the preset channel 300 is in the first state. It should be noted that the specific implementation of the preset channel 300 being arranged in the base 210 may be the similar as the specific implementation of the preset channel 300 being arranged in the connecting shaft 110 in the above embodiments, which is not repeated herein.

In some embodiments, the preset channel 300 may also be arranged at the coupling site between the connecting shaft 110 and the base 210. In some embodiments, the accommodating channel and the preset channel 300 overlap at the coupling site between the connecting shaft 110 and the base 210 to form the overlap area. When the center rod 130 is placed into the accommodating channel, extends to the coupling site between the connecting shaft 110 and the base 210 and occupies at least a part of the overlap area, the preset channel 300 is in the first state.

In some embodiments, the preset channel 300 has a cross-sectional shape in the first state different from a cross-sectional shape of the preset channel 300 in the second state. For example, the preset channel 300 is arranged at the coupling site between the connecting shaft 110 and the base 210. When the connecting shaft 110 is coupled to the base 210, the preset channel 300 is in the first state and is closed, and the closed preset channel 300 matches the second segment 122 in size, thereby constraining the control wire 120. When the connecting shaft 110 is detached from the base 210, the preset channel 300 is in the second state and is non-closed, i.e., the preset channel 300 has a gap that allows the second segment 122 to disengage.

In this way, when the preset channel 300 is in the first state, the first segment 121 and the third segment 123 cannot pass through the preset channel 300, and the second segment 122 is constrained within the preset channel 300; and when the preset channel 300 is in the second state, the second segment 122 can be separated from the preset channel 300 by the gap in the preset channel 300, so that the control wire 120 is detached from the constraint of the preset channel 300.

It is to be noted that the preset channel 300 being closed refers to that a shape of a cross-section of the preset channel 300 is closed, and the preset channel 300 being non-closed refers to that the shape of the cross-section of the preset channel 300 is divided into multiple non-enclosed portions.

In some embodiments, the connecting shaft 110 has a first contact surface 111, the base 210 has a second contact surface 211 configured to match the first contact surface 111. The first contact surface 111 is provided with a first through-slot 112, and the second contact surface 211 is provided with a second through-slot 212. When the connecting shaft 110 is coupled to the base 210, the first contact surface 111 and the second contact surface 211 fit together, and the first through-slot 112 and the second through-slot 212 together form a closed preset channel 300. When the connecting shaft 110 is detached from the base 210, the first contact surface 111 and the second contact surface 211 are separated from each other so that the preset channel 300 is non-closed.

Referring to FIG. 10, in one example, the first contact surface 111 includes a first distal blocking surface 1111, a first inclined surface 1112, and a first proximal blocking surface 1113 arranged in sequence from distal to proximal. The second contact surface 211 includes a second distal blocking surface 2111, a second inclined surface 2112 and a second proximal blocking surface 2113 arranged in sequence from distal to proximal. The first distal blocking surface 1111 may be fitted to the second distal blocking surface 2111. The first proximal blocking surface 1113 may be fitted to the second proximal blocking surface 2113. The first beveled surface 1112 may be at least partially fitted to the second beveled surface 2112. A maximum vertical distance h1 from an edge of the connecting shaft 110 to the first inclined surface 1112 becomes smaller as an axial distance from the first beveled surface 1112 to the first distal blocking surface 1111 becomes larger. For example, the first inclined surface 1112 is configured such that the maximum vertical distance h1 from the edge of the connecting shaft 110 to the first inclined surface 1112 gradually decreases from the distal end of the first inclined surface 1112 to the proximal end of the first inclined surface 1112. A maximum vertical distance h2 from an edge of the base 210 to the second inclined surface 2112 becomes smaller as an axial distance from the second inclined surface 2112 to the second proximal blocking surface 2113 becomes larger. For example, the second inclined surface 2112 is configured such that the maximum vertical distance h2 from the edge of the base 210 to the second inclined surface 2112 gradually decreases from the proximal end of the second inclined surface 2112 to the distal end of the second inclined surface 2112. Here, the maximum vertical distance from the edge of the connecting shaft 110 to the first inclined surface 1112 refers to, in the same cross-section, the largest one of all vertical distances between points on the edge of the connecting shaft 110 and an intersection line formed by the intersection of the first inclined surface 1112 and the cross-section. The maximum vertical distance from the edge of the base 210 to the second inclined surface 2112 refers to, in the same cross-section, the largest one of all vertical distances between points on the edge of the base 210 and an intersection line formed by the intersection of the second inclined surface 2112 and the cross-section. In this way, the contact portions of the second contact surface 211 and the first contact surface 111 make the connecting shaft 110 move radially with respect to the base 210 when the connecting shaft 110 moves axially away from the base 210. Therefore, the constraint of the connecting shaft 110 moving axially relative to the base 210 can be achieved by only constraining of the connecting shaft 110 moving radially relative to the base 210, making it easier to implement the coupling between the connecting shaft 110 and the base 210. In combination with the above embodiments, the coupling of the connecting shaft 110 to the base 210 can be achieved by using the center rod 130 to keep the connecting shaft 110 stationary relative to the base 210 at least in the radial direction of the connecting shaft 110. Further, the first through-slot 112 is arranged on the first inclined surface 1112 and is adjacent to the first proximal blocking surface 1113. The second through-slot 212 is arranged on the second inclined surface 2112 and is adjacent to the second proximal blocking surface 2113. The first through-slot 112 includes a first slot wall and a first slot opening. The second through-slot 212 includes a second slot wall and a second slot opening. The second segment 122 can be separated from the first through-slot 112 via the first slot opening and/or can be separated from the second through-slot 212 via the second slot opening. The first slot wall and the second slot wall each have a curved cross-section. Further, the first slot wall and the second slot wall each have a circular arc shaped cross-section. When the connecting shaft 110 is coupled to the base 210, the first distal blocking surface 1111 is fitted to the second distal blocking surface 2111, the first inclined surface 1112 is fitted to the second inclined surface 2112, and the first proximal blocking surface 1113 is fitted to the second proximal blocking surface 2113. The first through-slot 112 and the second through-slot 212 form a closed preset channel 300.

In this way, when the first slot opening of the first through-slot 112 is fitted to the second slot opening of the second through-slot 212, the first slot wall of the first through-slot 112 and the second slot wall of the second through-slot 212 form a sidewall of the closed preset channel 300. When the connecting shaft 110 is detached from the base 210, the first through-slot 112 is separated from the second through-slot 212 so that the first slot of the first through-slot 112 is no longer fitted to the second slot of the second through-slot 212, and the first slot wall of the first through-slot 112 and the second slot wall of the second through-slot 212 forming the sidewall of the closed preset channel 300 are separated from each other. Thus, the preset channel 300 is in a non-closed state, and the second segment 122 can be separated from the preset channel 300.

In some embodiments, the connecting shaft 110 has a first contact surface 111, the base 210 has a second contact surface 211 configured to match the first contact surface 111, and one of the first contact surface 111 and the second contact surface 211 is provided with a third through-slot 113. When the connecting shaft 110 is coupled to the base 210, the first contact surface 111 is fitted to the second contact surface 211 partially, a slot wall of the third through-slot 113 and a corresponding part of the other one of the first contact surface 111 and the second contact surface 211 together form the closed preset channel 300. When the connecting shaft 110 is detached from the base 210, the first contact surface 111 and the second contact surface 211 are separated from each other so that the preset channel 300 is non-closed. The difference from the above embodiments is that only the first contact surface 111 or the second contact surface 211 is provided with a through-slot.

Referring to FIG. 11, in one example, the first contact surface 111 includes a third distal blocking surface 1114, a third inclined surface 1115, and a third proximal blocking surface 1116 arranged in sequence from distal to proximal, and the second contact surface 211 includes a fourth distal blocking surface 2114, a fourth inclined surface 2115, a transition surface 2117, and a fourth proximal blocking surface 2116 arranged in sequence from distal to proximal. The transition surface 2117 is disposed between the fourth inclined surface 2115 and the fourth proximal blocking surface 2116. The third distal blocking surface 1114 may be fitted to the fourth distal blocking surface 2114. The third proximal blocking surface 1116 may be fitted to the fourth proximal blocking surface 2116. The third inclined surface 1115 may be at least partially fitted to the fourth inclined surface 2115. A maximum vertical distance h3 from an edge of the connecting shaft 110 to the third inclined surface 1115 becomes smaller as an axial distance from the third inclined surface 1115 to the third distal blocking surface 1114 becomes larger. For example, the third inclined surface 1115 is configured such that the maximum vertical distance h3 from the edge of the connecting shaft 110 to the third inclined surface 1115 gradually decreases from the distal end of the third inclined surface 1115 to the proximal end of the third inclined surface 1115. A maximum vertical distance h4 from an edge of the base 210 to the fourth inclined surface 2115 becomes smaller as an axial distance from the fourth inclined surface 2115 to the fourth proximal blocking surface 2116 becomes larger. For example, the fourth inclined surface 2115 is configured such that the maximum vertical distance h4 from the edge of the base 210 to the fourth inclined surface 2115 gradually decreases from the proximal end of the fourth inclined surface 2115 to the distal end of the fourth inclined surface 2115. The third through-slot 113 is arranged adjacent to the third proximal blocking surface 1114 on the third inclined surface 1115 to correspond to the transition surface 2117. The third through-slot 113 includes a third slot wall and a third slot opening. The second segment 122 can be separated from the third through-slot 113 via the third slot opening. The third slot wall has a curved cross-section. Further, the third slot wall has a circular arc shaped cross-section. When the connecting shaft 110 is coupled to the base 210, the third distal blocking surface 1114 is fitted to the fourth distal blocking surface 2114, the third inclined surface 1115 is fitted to the fourth inclined surface 2115, and the third proximal blocking surface 1116 is fitted to the fourth proximal blocking surface 2116. The third through-slot 113 and the transition surface 2117 form the preset channel 300 in a closed state.

In this way, when the third slot opening of the third through-slot 113 is fitted to the transition surface 2117, the third slot wall of the third through-slot 113 and the transition surface 2117 form a side wall of the preset channel 300 in a closed state. When the connecting shaft 110 is detached from the base 210, the third through-slot 113 is separated from the transition surface 2117 so that the third slot opening of the third through-slot 113 is no longer fitted to the transition surface 2117, and the third slot opening of the third through-slot 113 and the transition surface 2117 forming a sidewall of the preset channel 300 are separated from each other. Thus, the preset channel 300 is in a non-closed state, and the second segment 122 can be separated from the preset channel 300.

In some embodiments, the transition surface 2117 in FIG. 11 may be arranged on the first contact surface 111. For example, the transition surface 2117 is arranged between the third inclined surface 1115 and the third proximal blocking surface 1116. In this case, the third through-slot 113 may be arranged on the second contact surface 211, for example, arranged between the fourth inclined surface 2115 and the fourth proximal blocking surface 2116. That is, the positions of the transition surface 2117 and the third through-slot 113 in FIG. 11 may be interchanged, and the transition surface is disposed corresponding to the third through-slot after the position interchange. When the connecting shaft 110 is coupled to the base 210, a fourth through-slot and the transition surface form a closed preset channel 300, and when the connecting shaft 110 is detached from the base 210, the fourth through-slot is separated from the transition surface, and the preset channel 300 is non-closed.

In some embodiments, the connecting shaft 110 includes a first coupling portion 114, the base 210 includes a second coupling portion 213, and the first coupling portion 114 or the second coupling portion 213 is disposed axially biased. When the first coupling portion 114 or the second coupling portion 213 is driven to be arranged axially, the first coupling portion 114 is coupled to the second coupling portion 213, the first contact surface 115 is arranged on a side of the first coupling portion 114 facing the second coupling portion 213, and the second contact surface 214 is arranged on a side of the second coupling portion 213 facing the first coupling portion 114. In this embodiment, similar to the above embodiments, a number of through-slots forming the preset channel 300 may be two, and the two through-slots are respectively arranged on the first contact surface 115 and the second contact surface 214; and the number of through-slots forming the preset channel 300 may also be one, and the through-slot is arranged on the first contact surface 115 or the second contact surface 214.

In some embodiments, one of the first coupling portion 114 and the second coupling portion 214 includes a protrusion 116, and the other one of the first coupling portion 114 and the second coupling portion 214 includes a recess 215. The first coupling portion 114 or the second coupling portion 213 is disposed axially biased so that the protrusion 116 is separated from the recess 215. When the first coupling portion 114 or the second coupling portion 213 is driven to be disposed axially, the protrusion 116 is accommodated in the recess 215.

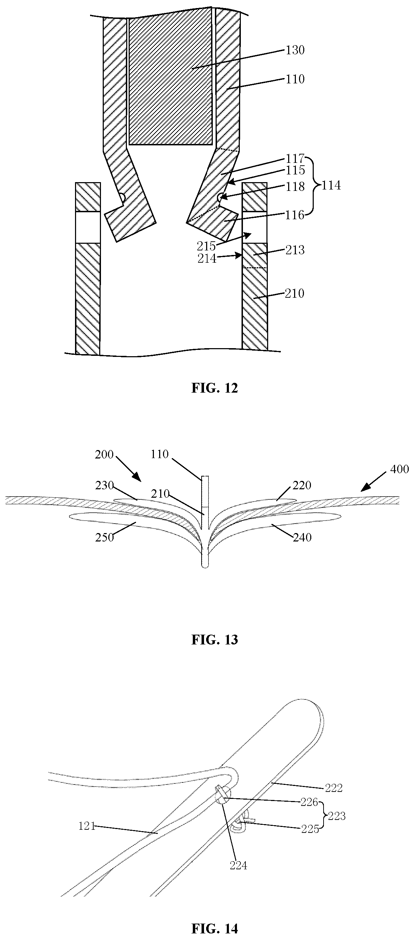

A description is given by taking as an example that one through-slot forming the preset channel 300 and is arranged on the first contact surface 115. As shown in FIG. 12, in one example, the first coupling portion 114 further includes an inwardly biased support member 117 and a fifth through-slot 118, the protrusion 116 is arranged at a distal end of the support member 117 and extends in a direction perpendicular to an axial direction of the support member 117, the fifth through-slot 118 is arranged on the support member 117 and includes a fifth slot opening and a fifth slot wall, and the recess 215 is disposed on an inner wall of the base 210. The delivery apparatus 100 further includes a center rod 130 movably arranged inside the connecting shaft 110. When the first coupling portion 114 needs to be coupled to the second coupling portion 213, the center rod 130 is moved distally to pass through the first coupling portion 114 and the second coupling portion 213 so that the support member 114 is driven to deflect until an axis of the support member 114 is parallel to an axis of the connecting shaft 110 and the protrusion 116 is accommodated in the recess 215. In this case, the fifth through-slot 118 and an inner wall of the second coupling portion 213 form a closed preset channel 300, i.e., the fifth slot opening 118 is fitted to the inner wall of the second coupling portion 213, and the fifth slot wall 118 and the inner wall of the second coupling portion 213 form a sidewall of the preset channel 300. When the connecting shaft 110 is detached from the base 210, the fifth through-slot 118 is separated from the inner wall of the second coupling portion 213, and the preset channel 300 is non-closed.

In some embodiments, the prosthesis includes a movable component, i.e., a first movable component. By controlling the movement of the first movable component through the first segment 121, a change in state of the prosthesis can be achieved. The "change in the state" herein may be a change in the structure, size or shape of the whole or a part of the prosthesis. For example, the prosthesis, such as a covered stent used for treating aortic stenosis, changes from a compressed state to an expanded state. A restraining wire (i.e., the first movable component) is generally used to restrain the covered stent in the compressed state in the delivery process, and upon the covered stent is positioned at a target position, the restraining wire is withdrawn from the covered stent by means of a control wire, so as to cause the covered stent to be changed from the compressed state to the expanded state. The "change in the state" herein may also refer to the implementation of the unlocked state/locked state between the prosthesis and the target tissue, between the prosthesis and the delivery system, and between the internal components of the prosthesis, and/or the mutual conversion between the locked state and the unlocked state. For example, when the left atrial appendage occluder is delivered, the control wire and the left atrial appendage occluder are in a locked state. Upon release of the left atrial appendage occluder, the control wire is driven to detach from the left atrial appendage occluder so that the left atrial appendage occluder is in an unlocked state. The "change in the state" may also be a change in position and/or attitude of the whole or of a part (e.g., the first movable component) of the prosthesis. For example, for a mitral valve clip used for treating mitral valve regurgitation, the relative rotation of a gripper (i.e., the first movable member) in the mitral valve clip is controlled by a control wire to achieve the clamping of mitral valve leaflets.

In some embodiments, the prosthesis includes two movable components, i.e., a first movable component and a second movable component. The first movable component is controlled by the first segment 121, and the second movable component is controlled by the third segment 123 to achieve the change in state of the prosthesis. When it is necessary to separate the delivery apparatus 100 from the prosthesis, the preset channel 300 may be made to be in a second state so that the control wire 120 can be disengaged from the preset channel 300 to facilitate extraction of the control wire 120 from the human body to separate the control wire 120 from the prosthesis. In this way, it is possible to control the movement of two movable components (i.e., the first movable component and the second movable component) of the prosthesis by means of one control wire 120.

In some embodiments, the prosthesis is a mitral valve clip 200 for treating mitral valve regurgitation. It should be noted that the prosthesis being the mitral valve clip 200 herein is merely exemplary, and in other embodiments, the prosthesis may not be the mitral valve clip 200. The present disclosure does not limit the specific type of the prosthesis.

As shown in FIG. 13, the mitral valve clip 200 includes a base 210, a gripper (i.e., a movable component), and an arm. The gripper and the arm are rotatable about an axis of the base 210. The gripper is disposed at a proximal end of the base 210, and the arm is disposed at a distal end of the base 210. In this embodiment, the gripper includes a first gripper 220 (i.e., a first movable component) and a second gripper 230 (i.e., a second movable component), which are arranged symmetrically about the axis of the base 210.

It should be noted that the first movable component being the first gripper 220 and the second movable component being the second gripper 230 herein are merely exemplary in the case where the prosthesis is the mitral valve clip 200. In other embodiments, the first movable component may not be the first gripper 220, and the second movable component may not be the second gripper 230. The present disclosure does not limit specific types of the first movable component and the second movable component.

Similarly, the arm includes a first arm 240 and a second arm 250 that are arranged symmetrically about the axis of the base 210 and located in a plane which the first gripper 220 and the second gripper 230 are defined to better clamp edges of anterior and posterior leaflets of a mitral valve 400. "The plane which the first gripper 220 and the second gripper 230 are defined" herein refers to a plane formed by an axis of the first gripper 220 and an axis of the second gripper 230. "The first arm 240 and the second arm 250 are located in the plane which the first gripper 220 and the second gripper 230 are defined" herein refers to that axes of both the first arm 240 and the second arm 250 are located in the plane which defined by the first gripper 220 and the second gripper 230. After the mitral valve clip 200 is delivered to a mitral valve target position, the anterior and posterior leaflets of the mitral valve 400 are clamped between the grippers and arms by driving the rotation of the grippers and arms to achieve treatment of mitral valve regurgitation. This also applies to treatment of tricuspid regurgitation, with the mitral valve clip replaced by a tricuspid valve clip.

Referring to FIGS. 1, 2, and 15, in one example, the first gripper 220 and the second gripper 230 are both sheet-like, and the first gripper 220 is resiliently connected to the second gripper 230 by a gripper-transition portion 102. The transition portion 102 is disposed at a distal end of the base 210. In an embodiment, the first gripper 220, the transition portion 102 and the second gripper 230 are integrally molded to form an inverted Ω-like shape. The first gripper 220 and the second gripper 230 are naturally in an extended state. As shown in FIG. 2, when the first segment 121 is pulled, the first gripper 220 is driven to rotate to move upwardly and toward the base 210, and the first gripper 220 is elastically deformed. When the first segment 121 is no longer pulled, the first gripper 220 is rotated to move away from the base 210 and return to its original shape. Similarly, when the third segment 123 is pulled, the second gripper 230 is driven to rotate to move upwardly and toward the base 210, and the second gripper 230 is elastically deformed. When the third segment 123 is no longer pulled, the second gripper 230 is rotated to move away from the base 210 and return to its original shape.

With continued reference to FIGS. 1 and 2, the first arm 240 and the second arm 250 are each pivotably coupled to the base 210. Further, the first arm 240 and the second arm 250 can be rotatably coupled to the base 210 via the same rotation shaft. In some embodiments, the first arm 240 has a U-shaped cross-section in a direction perpendicular to an axial direction of the first arm 240 to better wrap a first mitral valve leaflet edge 401 with the first gripper 220. Similarly, the second arm 250 has a U-shaped cross-section in a direction perpendicular to an axial direction of the second arm 250 to better wrap a second mitral valve leaflet edge 402 with the second gripper 230. When the mitral valve clip 200 clamps the mitral valve leaflets, the first arm 240 and the second arm 250 are driven to rotate and squeeze the edges of the mitral valve leaflets with the first gripper 220 and the second gripper 230 respectively, thereby achieving the clamping of the mitral valve leaflets by the mitral valve clip 200.

This embodiment has no particular limitation on the manner in which the movement of the first movable component is controlled by the first segment 121 and the movement of the second movable component is controlled by the third segment 123. For the mitral valve clip 200, it is sufficient that the first segment 121 is movably connected to the first gripper 220. In this way, on the one hand, the first gripper 220 can rotate with the movement of the first segment 121; and on the other hand, when the delivery apparatus 100 needs to be separated from the mitral valve clip 200, the control wire 120 can be separated from the first gripper 220. The third segment 123 is similarly related to the second gripper 230, which is not repeated herein.

In some embodiments, as shown in FIGS. 2 and 3, the first segment 121 extends distally from the preset channel 300, and after extending to the first gripper 220, inversely extends toward the proximal end of the delivery apparatus 100. In this way, the first segment 121 forms a curved portion near the first gripper 220, and the curved portion is interpenetrated with the first gripper 220 or an attachment disposed on the first gripper 220 to facilitate control of rotation of the first gripper 220.

In this embodiment, both second segment 122 and third segment 123 are configured to be detachable from the first gripper 220 so that when the control wire 120 need to be withdrawn from the first gripper 220, it can be ensured that the third segment 123 and the second segment 122 can be withdrawn from the first gripper 220.

In some embodiments, a junction between the third segment 123 and the second segment 122 is a first transition portion 124, and an area of a cross-section of the first transition 124 in a direction from the second segment 122 to the third segment 123 gradually increases. Thus, a size of the second segment 122 is smaller than or equal to the first size so that the second segment 122 can be placed in the preset channel 300 in the first state, and a size of at least the junction of the third segment 123 to the second segment 122 is larger than the first size so that the third segment 123 cannot pass through the preset channel 300 in the first state. In addition, the junction between the third segment 123 and the second segment 122 is the first transition portion 124 with a smooth transition, so that there is no significant step at the junction between the third segment 123 to the second segment 122.

During the process of withdrawing the first control wire 120, the entire first control wire 120 needs to be detached from the first clamping arm 220. In this way, the second segment 122 and third segment 123 can smoothly and sequentially detach from the first clamping arm 220, avoiding any impact on the posture and/or position of the first clamping arm 220. Furthermore, by providing the first transition portion 124 with the smooth transition, during the process of withdrawing the control wire 120, the control wire 120 can be prevented from be jammed on an other component of the prosthesis system, and the control wire 120 can also be prevented from being worn out, thereby reducing the possibility of the control wire 120 to break.

Referring to FIG. 14, in some embodiments, the attachment described above is a retaining wire 223. The retaining wire 223 is disposed on the first gripper 220 by tying, knotting, or the like, and forms a body 226 for interpenetration with the curved portion of the first segment 121.

The body 226 is annular to form a first through hole for interpenetration with the curved portion as described above, and the retaining wire 223 further has a retaining knot 225 connected to the body 226 and disposed on the first gripper 220. That is, the retaining wire 223 forms a knot-like structure. The curved portion of the first segment 121 passes through the first through hole to be interpenetrated with the body 226.

Further, the first gripper 220 is provided with a second through hole 224 on a surface of the first gripper 220, the body 226 passes through the second through hole 224 from a side of the first gripper 220 away from the base 210, and the retaining knot 225 is disposed on the side of the first gripper 220 away from the base 210 and is not able to pass through the second through hole 224.

In some embodiments, the retaining knot 225 of the retaining wire 223 is wound around the second through hole 224 to securely connect with the first gripper 220.

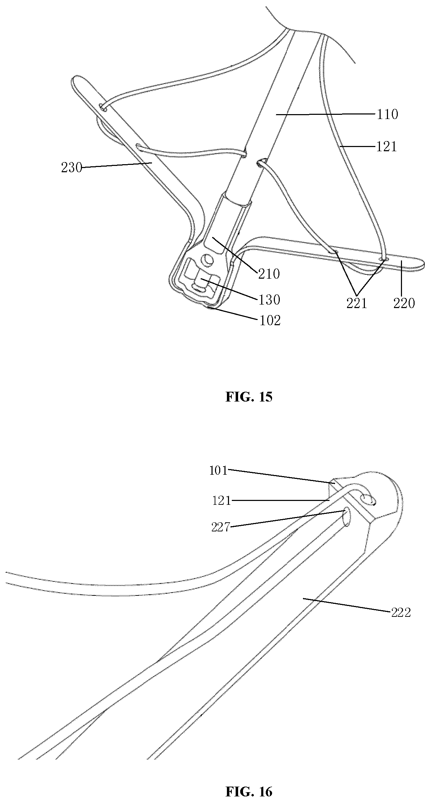

Referring to FIG. 15, in yet other embodiments, the first gripper 220 is provided with two third through holes 221, and an end of the first segment 121 away from the second segment 122 (i.e., an end of the curved portion) passes through one third through hole 221 from a side of the first gripper 220 away from the first arm 240, and then passes through the other third through hole 221 from a side of the first gripper 220 facing the first arm 240. In this way, it is possible to interpenetrate the curved portion of the first segment 121 with the first gripper 220.

In some embodiments, the first gripper 220 is provided with an even number of more than two third through holes 221, through which the end of the first segment 121 away from the second segment 122 (i.e., an end of the curved portion) alternatingly passes. In this way, it is also possible to interpenetrate the curved portion of the first segment 121 with the first gripper 220.

Referring to FIG. 16, in some embodiments, the first gripper 220 is provided with a protrusion 101 having a fourth through hole 227. The end of the first segment 121 away from the second segment 122 (i.e., an end of the curved portion) passes through the fourth through hole 227. In this way, it is also possible to interpenetrate the curved portion of the first segment 121 with the first gripper 220. In some embodiments, the fourth through hole 227 has the same extension direction as the first gripper 220.

It should be noted that the connection between the third segment 123 and the second gripper 230 can be referred to the connection between the first segment 121 and the first gripper 220 as described above, which is not be repeated herein. In some embodiments, a junction between the first segment 121 and the second segment 122 may be a second transition portion 125 having the similar function as the first transition portion 124, which is not be repeated herein.

In another embodiments, both the first segment 121 and the second segment 122 may be configured to be detachable from the second gripper 230, so that when the control wire 120 need to be withdrawn from the second gripper 230, it can be ensured that both first segment 121 and second segment 122 can be withdrawn from the second gripper 230.

In some embodiments, the mitral valve clip 200 further includes: an actuation assembly configured to control rotation of the first arm 240 and the second arm 250.

With continued reference to FIGS. 1 and 2, the actuation assembly includes a first connecting rod 261, a second connecting rod 262, and a slider 263. The first connecting rod 261 has a proximal end rotatably coupled to the first arm 240 and a distal end rotatably coupled to the slider 263 And the slider 263 is movable in an axial direction of the base 210.Thus, the first connecting rod 261, the first arm 240, the base 210 and the slider 263 form a kinematic mechanism similar to a crank-slider mechanism. When the slider 263 is driven close to or away from the base 210, the first arm 240 is driven to undergo rotational movement. Similarly, the second connecting rod 262 has a proximal end rotatably coupled to the second arm 250 and a distal end rotatably coupled to the slider 263.Thus, the second connecting rod 262, the second arm 250, the base 210 and the slider 263 form a kinematic mechanism similar to a crank-slider mechanism. When the slider 263 is driven close to or away from the base 210, the second arm 250 is driven to undergo rotational movement.

In some embodiments, the slider 263 is T-shaped, i.e., the slider 263 includes a horizontal portion and a vertical portion. The base 210 is hollow, and the vertical portion of the slider 263 is movably housed within the base 210. Two ends of the horizontal portion of the slider 263 are rotationally connected to the first connecting rod 261 and the second connecting rod 262 respectively, and the vertical portion of the slider 263 is removably connected to the center rod 130, for example, by a threaded connection. In this way, the slider 263 can be driven by the center rod 130 to move to rotate the first arm 240 and the second arm 250. Further, the first connecting rod 261 and the second connecting rod 262 are arranged symmetrically about the axis of the base 210 so that when the first arm 240 and the second arm 250 are driven by the slider 263 to move, the first arm 240 and the second arm 250 have the same rotation angle when rotated.

In some embodiments, the delivery apparatus 100 further includes a catheter 140 sleeved to the connecting shaft 110. The catheter 140 is provided with two second passages extending in an axial direction of the catheter 140. The end of the first segment 121 away from the second segment 122 extends through one second channel from a distal end of the one second channel to a proximal end of the one second channel, and the end of the third segment 123 away from the second segment 122 extends through the other second channel from a distal end of the other second channel to a proximal end of the other second channel. In this way, it may be convenient for an operator to pull, at a proximal end of the catheter 140, the end of the first segment 121 away from the second segment 122 to adjust the rotation angle of the first gripper 220, and for an operator to pull, at the proximal end of the catheter 140, the end of the third segment 123 away from the second segment 122 to adjust the rotation angle of the second gripper 230. When it is necessary to separate the control wire 120 from the mitral valve clip 200, the preset channel is adjusted to the second state, and the operator pulls, at the proximal end of the catheter 140, the end of the first segment 121 away from the second segment 122 or the end of the third segment 123 away from the second segment 122 to achieve separation of the control wire 120 from the mitral valve clip 200.

It is to be noted that the present disclosure does not limit the specific implementations of removably coupling the connecting shaft 110 to the base 210. In addition to the manners described in the above embodiments, any other suitable manner may be adopted, such as the manners illustrated in figures 5A, 5B, 6A and 6B of the accompanying drawings disclosed in U.S. Patent Document US20210145574A1, and the manners illustrated in figures 82A, 82B and 84 of the accompanying drawings disclosed in Chinese Patent Document CN102395331B.

With continued reference to FIGS. 1 to 19, other embodiments of the present disclosure provide a delivery apparatus 100 configured to deliver a prosthesis. The prosthesis includes a base 210 and a first movable component. The delivery apparatus 100 includes a connecting shaft 110 and a control wire 120. The connecting shaft 110 is detachably coupled to the base 210. A preset channel 300 is provided in the connecting shaft 110 at an angle relative to an axis of the connecting shaft 110, or provided at a coupling site between the connecting shaft 110 and the base 210 at an angle relative to an axis of the connecting shaft 110. The control wire 120 includes a first segment 121, a second segment 122 and a third segment 123 connected successively. At least a part of each of the first segment 121 and the third segment 123 connected to the second segment 122 has a size larger than a size of the second segment 122. The first segment 121 is configured to independently drive the first movable component. The second segment 122 is accommodatable within the preset channel 300. The preset channel 300 has a first state and a second state. In response to the preset channel 300 being in the first state, the first segment 121 and the third segment 123 are configured to cause the second segment 122 to be constrained within the preset channel 300, and in response to the preset channel 300 being in the second state, the control wire 120 can be separated from the preset channel 300.

In fact, the delivery apparatus 100 in this embodiment has the similar structure and beneficial effects as the partial delivery apparatus 100 in the above-described other embodiments (the partial delivery apparatus 100 here refers to the delivery apparatus 100 with the preset channel arranged in the connecting shaft 110, or the delivery apparatus 100 with the preset channel arranged at the coupling site between the connecting shaft 110 and the base 210), which is not repeated herein.

It is understood by those of ordinary skill in the art that the above embodiments are specific embodiments for realizing the present disclosure, and that in practical application, various changes in form and detail can be made without deviating from the spirit and scope of the present disclosure. Any person skilled in the art, without departing from the spirit and scope of the present disclosure, may make various changes and modifications. Therefore, the protection scope of the present disclosure should be subject to the scope defined by the appended claims.

## Claims

1. A prosthesis system, comprising:
a delivery apparatus, including a connecting shaft and a control wire; and
a prosthesis, including a base and a first movable component, wherein the base is detachably coupled to the connecting shaft;
wherein a preset channel is provided in at least one of the connecting shaft and the base at an angle relative to an axis of the connecting shaft;
wherein the control wire includes a first segment, a second segment and a third segment connected successively, at least a part of each of the first segment and the third segment connected to the second segment has a size larger than a size of the second segment, the first segment is configured to independently drive the first movable component, and the second segment is accommodatable within the preset channel; and
wherein the preset channel has a first state and a second state, in response to the preset channel being in the first state, the first segment and the third segment are configured to cause the second segment to be constrained within the preset channel, and in response to the preset channel being in the second state, the control wire is separable from the preset channel.

2. The prosthesis system according to claim 1, wherein:
in response to the preset channel being in the first state, a part of the preset channel configured to accommodate the second segment has a first size, and the first size is smaller than the size of the part of each of the first segment and the third segment connected to the second segment and larger than or equal to the size of the second segment; and
in response to the preset channel being in the second state, the part of the preset channel configured to accommodate the second segment has a second size, and the second size is larger than a size of each of at least one of the first segment and the third segment.

3. The prosthesis system according to claim 2, wherein:
the delivery apparatus further includes a center rod;
at least one of the connecting shaft and the base is provided with an accommodating channel arranged in an axial direction of the connecting shaft, wherein the accommodating channel intersects the preset channel, and an area where the accommodating channel overlaps the preset channel is an overlap area;
when the center rod is placed into the accommodating channel and occupies at least a part of the overlap area, the preset channel is in the first state, and a size of a part of the remaining area of the preset channel configured to accommodate the second segment is the first size; and
when the center rod is positioned outside the overlap area, the preset channel is in the second state, and a size of the part of the preset channel configured to accommodate the second segment is the second size.

4. The prosthesis system according to claim 3, wherein:
a size of a part of the overlap area occupied by the center rod is a third size, the second size is larger than or equal to a sum of the third size and the size of the second segment, and the second size is smaller than a sum of the third size and the size of the part of each of the first segment and the third segment connected to the second segment.

5. The prosthesis system according to claim 3, wherein:
the preset channel is arranged in the connecting shaft;
the accommodating channel includes a first accommodating segment arranged in the connecting shaft, and an area where the accommodating segment overlaps the preset channel is the overlap area; and
when the center rod is placed into the first accommodating segment and occupies at least the part of the overlap area, the preset channel is in the first state.

6. The prosthesis system according to claim 3, wherein:
the preset channel is arranged in the base;
the accommodating channel includes a first accommodating segment on the connecting shaft, and a second accommodating segment on the base, wherein the first accommodating segment and the second accommodating segment are connected with each other when the base is detachably coupled to the connecting shaft, and an area where the second accommodating segment overlaps the preset channel is the overlap area; and
when the center rod is placed into the first accommodating segment and the second accommodating segment and occupies at least the part of the overlap area, the preset channel is in the first state.

7. The prosthesis system according to claim 3, wherein:
the preset channel is arranged at a coupling site between the connecting shaft and the base; and
the accommodating channel and the preset channel overlap at the coupling site to form the overlap area.

8. The prosthesis system according to any one of claims 3 to 7, wherein:
in response to a size of the overlap area being equal to the second size, the center rod occupies a part of the overlap area; and
in response to the size of the overlap area being smaller than the second size, the center rod occupies at least a part of the overlap area.

9. The prosthesis system according to claim 1, wherein:
the preset channel is arranged at a coupling site between the connecting shaft and the base;
when the connecting shaft is coupled to the base, the preset channel is in the first state and is closed; and
when the connecting shaft is detached from the base, the preset channel is in the second state and is non-closed.

10. The prosthesis system according to claim 9, wherein:
the connecting shaft has a first contact surface, wherein the first contact surface is provided with a first through-slot;
the base has a second contact surface configured to match the first contact surface, wherein the second contact surface is provided with a second through-slot;
when the connecting shaft is coupled to the base, the first contact surface and the second contact surface fit together, and the first through-slot and the second through-slot together form the preset channel that is closed; and
when the connecting shaft is detached from the base, the first contact surface and the second contact surface are separated from each other so that the preset channel is non-closed.

11. The prosthesis system according to claim 10, wherein:
the first contact surface includes a first distal blocking surface, a first inclined surface, and a first proximal blocking surface arranged in sequence from distal to proximal, and a maximum vertical distance from an edge of the connecting shaft to the first inclined surface becomes smaller as an axial distance from the first inclined surface to the first distal blocking surface becomes larger;
the second contact surface includes a second distal blocking surface, a second inclined surface and a second proximal blocking surface arranged in sequence from distal to proximal, and a maximum vertical distance from an edge of the base to the second inclined surface becomes smaller as an axial distance from the second inclined surface to the second proximal blocking surface becomes larger; and
the first through-slot is arranged on the first inclined surface, the second through-slot is correspondingly arranged on the second inclined surface, when the connecting shaft is coupled to the base, the first through-slot and the second through-slot together form the preset channel that is closed, and when the connecting shaft is detached from the base, the first through-slot is separated from the second through-slot so that the preset channel is non-closed.

12. The prosthesis system according to claim 9, wherein:
the connecting shaft has a first contact surface;
the base has a second contact surface configured to match the first contact surface;
one of the first contact surface and the second contact surface is provided with a third through-slot;
when the connecting shaft is coupled to the base, the first contact surface and the second contact surface fit together, a slot wall of the third through-slot and a corresponding part of the other one of the first contact surface and the second contact surface together form the preset channel that is closed; and
when the connecting shaft is detached from the base, the first contact surface and the second contact surface are separated from each other so that the preset channel is non-closed.

13. The prosthesis system according to claim 12, wherein:
the first contact surface includes a third distal blocking surface, a third inclined surface, and a third proximal blocking surface arranged in sequence from distal to proximal, and a maximum vertical distance from an edge of the connecting shaft to the third inclined surface becomes smaller as an axial distance from the third inclined surface to the third distal blocking surface becomes larger;
the second contact surface includes a fourth distal blocking surface, a fourth inclined surface, a transition surface, and a fourth proximal blocking surface arranged in sequence from distal to proximal, and a maximum vertical distance from an edge of the base to the fourth inclined surface becomes smaller as an axial distance from the fourth inclined surface to the fourth proximal blocking surface becomes larger;
the third through-slot is arranged on the third inclined surface to correspond to the transition surface; and
when the connecting shaft is coupled to the base, the third through-slot and the transition surface together form the preset channel that is closed, and when the connecting shaft is detached from the base, the third through-slot and the transition surface are separated from each other so that the preset channel is non-closed.

14. The prosthesis system according to claim 12, wherein:
the first contact surface includes a third distal blocking surface, a third inclined surface, a transition surface, and a third proximal blocking surface arranged in sequence from distal to proximal, and a maximum vertical distance from an edge of the connecting shaft to the third inclined surface becomes smaller as an axial distance from the third inclined surface to the third distal blocking surface becomes larger;
the second contact surface includes a fourth distal blocking surface, a fourth inclined surface, and a fourth proximal blocking surface arranged in sequence from distal to proximal, and a maximum vertical distance from an edge of the base to the fourth inclined surface becomes smaller as an axial distance from the fourth inclined surface to the fourth proximal blocking surface becomes larger;
the third through-slot is arranged on the fourth inclined surface to correspond to the transition surface; and
when the connecting shaft is coupled to the base, the third through-slot and the transition surface together form the preset channel that is closed, and when the connecting shaft is detached from the base, the third through-slot and the transition surface are separated from each other so that the preset channel is non-closed.

15. The prosthesis system according to any one of claims 1 to 14, wherein:
the prosthesis further includes a second movable component; and
the third segment is configured to independently drive the second movable component.

16. The prosthesis system according to claim 15, wherein the prosthesis is a mitral valve clip or a tricuspid valve clip.

17. The prosthesis system according to claim 16, wherein the first movable component is a first gripper, the second movable component is a second gripper, and the first gripper and the second gripper are rotatable about an axis of the base; and
wherein the first segment is movably connected to the first gripper, and the second segment is movably connected to the second gripper.

18. A delivery apparatus configured to deliver a prosthesis, wherein the prosthesis includes a base and a first movable component, and the delivery apparatus comprises:
a connecting shaft, detachably coupled to the base, wherein a preset channel is provided in the connecting shaft at an angle relative to an axis of the connecting shaft, or provided at a coupling site between the connecting shaft and the base at an angle relative to an axis of the connecting shaft; and
a control wire, including a first segment, a second segment and a third segment connected successively, at least a part of each of the first segment and the third segment connected to the second segment has a size larger than a size of the second segment, the first segment is configured to independently drive the first movable component, and the second segment is accommodatable within the preset channel;
wherein the preset channel has a first state and a second state, in response to the preset channel being in the first state, the first segment and the third segment are configured to cause the second segment to be constrained within the preset channel, and in response to the preset channel being in the second state, the control wire is separable from the preset channel.
